# EUROPÄISCHE PATENTSCHRIFT

(11) **EP 2 168 484 B1**
(45) Veröffentlichungstag und Bekanntmachung des Hinweises auf die Patenterteilung: **26.10.2011**
(21) Anmeldenummer: 09154842.0
(22) Anmeldetag: 11.03.2009
(51) Int. Cl.: A61B 6/00, A61B 6/03, A61B 6/04, A61B 19/00

(54) **Röntgengerät zur Brustuntersuchung mit einer in eine Patientenliege integrierten Gantry**
X-ray device for breast examination with a gantry integrated into a patient table
Dispositiv d'onde X pour examination des seins avec portique intégrée dans une table pour un patient

(30) Priorität: 29.09.2008 DE 102008042430
(43) Veröffentlichungstag der Anmeldung: 31.03.2010
(73) Patentinhaber: MIR Medical Imaging Research Holding GmbH, 91096 Möhrendorf (DE); Friedrich-Alexander-Universität Erlangen-Nürnberg, 91054 Erlangen (DE)
(72) Erfinder: Schilling, Harry, 85072, Eichstätt (DE); Kalender, Willi, 91096, Möhrendorf (DE)
(74) Vertreter: Lohr, Georg

(56) Entgegenhaltungen:
- EP-A- 1 864 611
- WO-A-98/49939
- WO-A-2004/043535
- WO-A-2008/054279
- DE-A1- 10 026 792
- US-A1- 2004 092 826
- US-A1- 2008 089 471
- US-A1- 2008 221 443
- US-B1- 6 298 114

## Beschreibung

### Technisches Gebiet

Die Erfindung betrifft ein Röntgengerät zur Abbildung der weiblichen Brust (Mammografie).

### Stand der Technik

Zur Untersuchung der weiblichen Brust sind verschiedene Röntgengeräte bekannt. Unter einer Liege, auf der eine zu untersuchende Patientin liegt, befindet sich eine Röntgenvorrichtung mit einer rotierenden Gantry, welche eine Röntgenröhre und einem Detektor aufweist. Ein solches Gerät ist beispielsweise in der US 4,015,836 offenbart. Nachteilig an diesem Stand der Technik ist der große Platzbedarf so wie die mangelnde Zugänglichkeit der zu untersuchenden Brust. Weiterhin wird die Patientin in eine relativ unbequeme Lage mit tief liegendem Kopf gebracht, damit von der Röntgenvorrichtung ein möglichst großer Anteil der Brust erfasst werden kann. Eine Verbesserung stellt hier die Anordnung aus der US 2006/0094950 dar. Die Patientin hat hier eine bequemere Lage. Weiterhin ist auch die zu untersuchende Brust nur mit speziellen Instrumenten zugänglich. Nachteilig an dieser Anordnung ist der große Platzbedarf, der sich durch die große Bauform der Gantry ergibt. In der US 2007/0064867 ist ein Röntgengerät basierend auf einem Spiral-CT Scanner offenbart. Hier wird die Auflösung durch die wenig stabile mechanische Konstruktion begrenzt. Zudem ist auch hier die zu untersuchende Brust nicht von außen zugänglich.

In der EP-A-1864 611 ist ein Brust-Röntgensystem offenbart, bei dem in einem CT-Scanner Vakuumaufnahmen zur Aufnahme der zu untersuchenden Brust vorgesehen sind.

Dokument EP1864611 offenbart ein Röntgengerät zur Abbildung einer Brust einer Patientin, mit einer Röntgeneinrichtung, umfassend eine Röntgenröhre und einen Röntgendetektor, und einer geneigt angeordneten Patientenliegefläche mit einem Brustausschnitt zur Aufnahme einer Patientin, mit einer um eine Drehachse drehbare Gantry mit der Röntgenröhre sowie dem Röntgendetektor, wobei die Gantry an der Patientenliege mechanisch steif befestigt ist und zur Abbildung der Brust in eine kontinuierliche Rotationsbewegung versetzt werden kann, und die Gantry mittels eines Gantryhubantriebs in eine feste Richtung bewegt werden kann, wobei diese Bewegung in Abhängigkeit von der Rotationsbewegung erfolgt.

In der US 2008/221443 A1 ist ein transportables Mammografiesystem offenbart, welches ein rotierendes Röntgengerät hat und während der Untersuchung Biopsien erlaubt.

### Darstellung der Erfindung

Der Erfindung liegt die Aufgabe zugrunde, ein Röntgengerät zu gestalten, welches die weibliche Brust diagnostisch exakt, schnell und kostengünstig und in einer bequemen Lage der Patientin abbildet.

Diese Aufgabe wird durch ein Röntgengerät nach Anspruch 1 gelöst. Vorteilhafte Ausgestaltungen der Erfindung sind in den Unteransprüchen angegeben.

Ein erfindungsgemäßes Röntgengerät zur Abbildung einer weiblichen Brust umfasst eine Patientenliege 20, an welcher eine Gantry 10 eines Spiral-Computertomographen mechanisch steif aufgehängt ist. Die Patientenliege 20 ist bevorzugt näherungsweise horizontal angeordnet und weist einen Brustausschnitt 21 auf, durch welchen die Brust 31 einer Patientin 30 vorzugsweise nach unten in Richtung der Gantry 10 hängt. Die Gantry 10 hat einen Gantryhubantrieb 11, mit dem sie gegenüber dem Patiententisch 20 bewegt werden kann. Zur Röntgenaufnahme rotiert die Gantry 10 um die Brust 31 der Patientin 30. Gleichzeitig und/oder in Zeitintervallen gestaffelt erfolgt eine Verschiebung der Gantry 10 in Längsrichtung der Brust, das heißt vorzugsweise in vertikaler Richtung. Die Verschiebung kann wahlweise kontinuierlich mit konstanter Geschwindigkeit oder proportional zur Rotation der Gantry erfolgen. Alternativ kann die Verschiebung auch in Schritten erfolgen, so dass beispielsweise nach jeder Umdrehung der Gantry ein Versatz um die Breite des Detektors 14 erfolgt.

Mit einem erfindungsgemäßen Röntgengerät sollen auch Röntgenaufnahmen mit einer Auflösung in der Größenordnung von 10 Mikrometer bis 500 Mikrometer, bevorzugt bis 100 Mikrometer möglich sein. Wie Untersuchungen gezeigt haben, können bei derartigen Auflösungen auch geringste mechanische Toleranzen und Schwankungen die Bildqualität erheblich beeinträchtigen. Bei einem Röntgengerät nach dem Stand der Technik wird die Gantry vor der Aufnahme in eine geeignete Aufnahmeposition gebracht. Nach dem Stillstand der Gantry vergehen einige Sekundenbruchteile bis Sekunden, in denen mechanische Schwingungen abklingen können, bis die Aufnahme ausgelöst wird. Bei einer Spiral-Computertomographen Gantry erfolgt jedoch gleichzeitig mit der Aufnahme eine kontinuierliche Rotation der Gantry um die Brust. Hier wirken sich mechanische Vibrationen und Toleranzen direkt auf die Bildqualität aus.

Wesentlich für die Erfindung ist daher eine mechanisch steife Verbindung zwischen der Gantry 10 und der Patientenliege 20. Diese ist notwendig, um bei einer hochauflösenden Abbildung Bewegungsartefakte durch mechanische Vibrationen und Lagetoleranzen der Gantry in Bezug auf die Brust zu minimieren. Entsprechend muss auch der Gantryhubantrieb 11 mechanisch steif ausgeführt sein. Mit einer solchen erfindungsgemäßen Anlage lassen sich Aufnahmen mit einer wesentlich höheren Qualität anfertigen, als mit Anlagen entsprechend dem Stand der Technik, bei denen der Patiententisch und die Röntgeneinrichtung separat aufgestellt beziehungsweise aufgehängt sind. Zudem beeinflussen auch mechanische Toleranzen oder eine eventuelle Durchbiegung der Patientenliege 20 nicht die Messgenauigkeit. Somit kann insbesondere mit dem Zentralstrahl des Strahlenfächers 16 im brustwandnahen Bereich die Auflösung optimiert werden. Dieser Zentralstrahl ist derjenige Strahl, der senkrecht auf den Detektor 14 auftrifft. Er liegt vorteilhafterweise möglichst nahe an der Brustwand der Patientin 30, um einen möglicht großen Bereich der Brust zu erfassen. Somit werden Röntgenröhre 15 und Detektor 14 bevorzugt so angeordnet, dass der Zentralstrahl des Strahlenfächers 16 möglichst an der Seite des Strahlenfächers liegt, welche der Patientenliege 20 zugewandt ist. Mit dem Zentralstrahl kann grundsätzlich die beste Auflösung erreicht werden. Durch die erfindungsgemäße Anordnung kann verhindert werden, dass Vibrationen und Lagetoleranzen der Gantry und anderer Komponenten des Röntgengerätes die hohe Abbildungsqualität im Zentralstrahl verringern.

Um die Genauigkeit des erfindungsgemäßen Röntgengerätes weiter zu verbessern, ist eine Fixiervorrichtung 40 für die Brust vorgesehen, welche ebenfalls mechanisch steif mit der Patientenliege verbunden ist. Aus hygienischen Gründen kann diese aber lösbar mit der Liege verbunden sein. Vorzugsweise arbeitet die Fixiervorrichtung 40 mithilfe eines Vakuums, das mit einer Vakuumpumpe 42 hergestellt wird.

Ein wichtiger Vorteil der Erfindung liegt weiterhin in dem äußerst kompakten Aufbau. So kann eine Spiral-Computertomographen Gantry wesentlich Platz sparender aufgebaut werden, als die bekannten Gantries, welche einen großflächigen Detektor benötigen, um die Brust als Ganzes abzubilden. Durch die spiralförmige Aufzeichnung kann deine erfindungsgemäßen Anordnung ein wesentlich kleinerer Detektor eingesetzt werden. Dadurch kann auch die Gantry wesentlich flacher gebaut werden. Die Höhe der Gantry liegt in einem Bereich von 5cm bis 20 cm, bevorzugt bei 10 cm. Aus diesem Grund und wegen der Aufhängung der Gantry an dem Tisch ist der Raum zwischen der Gantry 10 und dem darunter liegenden Fußboden frei zugänglich und kann somit von einer die Untersuchung durchführenden Person begangen werden. Ebenso können auch weitere Untersuchungsgeräte, wie Biopsiegeräte in diesem Raum angeordnet werden. Durch die freie Zugänglichkeit des Raumes unter der Gantry ist eine Therapie bei gleichzeitiger Bildaufnahme möglich.

Weiterhin können durch die Integration der Gantry 10 in die Patientenliege 20 Aufnahmen bis zur Unterkante der Patientenliege durchgeführt werden.

Besonders vorteilhaft ist es, wenn die Patientenliege 20 in der Höhe verstellbar ist. Hierzu wird bevorzugt einen Patientenliegenhubantrieb 22 eingesetzt. Da die Gantry 10 an der Patientenliege 20 aufgehängt ist, kann die Patientenliege grundsätzlich in der Höhe frei verstellt werden. Durch eine Höhenverstellung kann zusätzlicher Platz für weitere Untersuchungsgeräte oder sogar für eine aufrechte Arbeitsposition einer die Untersuchung durchführenden Person geschaffen werden.

In einer weiteren vorteilhaften Ausgestaltung der Erfindung weist das Röntgengerät Räder 23 auf, so dass es zwischen verschiedenen Einsatzorten bewegt werden kann. Ebenso ist hier eine exakte Justage des Gerätes in Bezug auf andere diagnostische Anlagen oder Geräte möglich. So kann es beispielsweise in Bezug auf ein anderes Röntgengerät, ein Ultraschallgerät oder auf eine Biopsieeinrichtung ausgerichtet werden. Da die Gantry 10 an dem Tisch aufgehängt ist, wird durch eine Verschiebung des Tisches mit der Gantry die Justage der Gantry in Bezug auf den Tisch nicht verändert. Somit bleiben die Bildqualität und auch die Lagegenauigkeit konstant.

Weiterhin ist es vorteilhaft, wenn die Patientenliege 20 kippbar gelagert ist.

Unter dem Begriff der mechanisch steifen Verbindung wird eine Verbindung verstanden, die durchaus lösbar sein kann, jedoch die zu verbindenden Teile derart miteinender verbindet, dass mechanische Bewegungen im Betrieb kleiner als die Auflösung des Röntgengerätes in der Größenordnung von 10 Mikrometer bis 500 Mikrometer, bevorzugt bis 100 Mikrometer sind.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt ein erfindungsgemäßes Röntgengerät
Figur 2 zeigt ein erfindungsgemäßes Röntgengerät mit einer fahrbaren Patientenliege
Figur 3 zeigt einen Schnitt durch ein erfindungsgemäßes Röntgengerät
Figur 4 zeigt schematisch einen Ausschnitt eines erfindungsgemäßen Röntgengerätes zur Erläuterung des Strahlenganges.
Figur 5 zeigt exemplarisch eine Fixiervorrichtung zur erfindungsgemäßen Kombination mit dem Röntgengerät

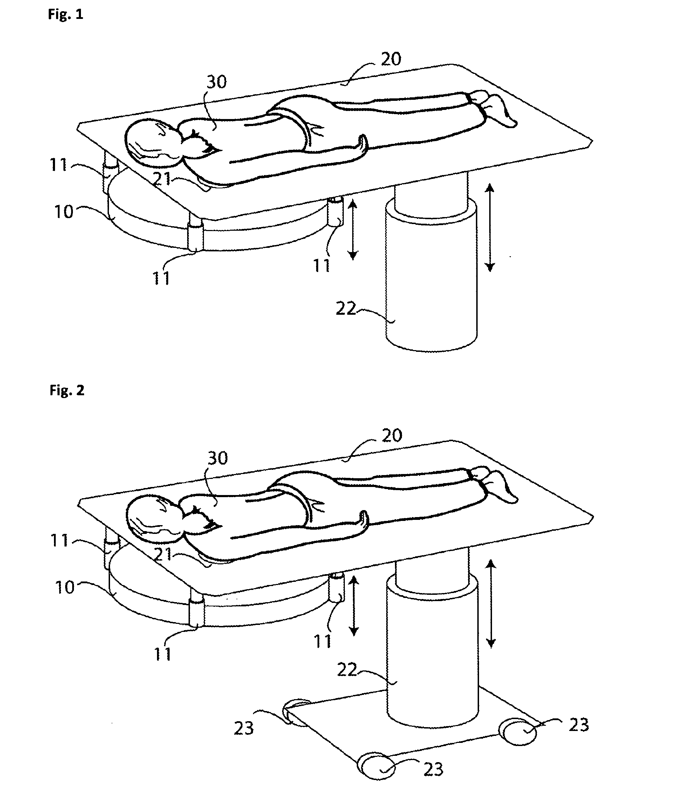

In Figur 1 ist ein erfindungsgemäßes Röntgengerät dargestellt. Auf der Patientenliege 20 liegt eine Patientin 30. Die zu untersuchende Brust hängt über einen Brustausschnitt 21 durch die Patientenliege 20 in den Aufnahmebereich einer Gantry 10. Die Gantry 10 ist eine Spiral-Computertomographen Gantry mit einer Röntgenröhre und einem Detektor, welche sich um die zu untersuchende Brust drehen. Während der Drehung wird die Brust abgebildet. Gleichzeitig mit der Drehung wird über den Gantryhubantrieb 11 eine Verschiebung in vertikaler Richtung durchgeführt, so dass die Brust spiralförmig abgetastet wird. Die Patientenliege 20 ist über einen Patientenliegenhubantrieb 22 in der Höhe verstellbar. Optional kann bei einem fest installierten Patiententisch dieser auch noch um die Achse des Patientenliegenhubantriebs 22 drehbar sein.

In Figur 2 ist eine weitere Ausgestaltung der Erfindung dargestellt. Hier weist die ganze Anordnung noch zusätzliche Räder 23 auf, um diese auf einfache Weise zu verschieben.

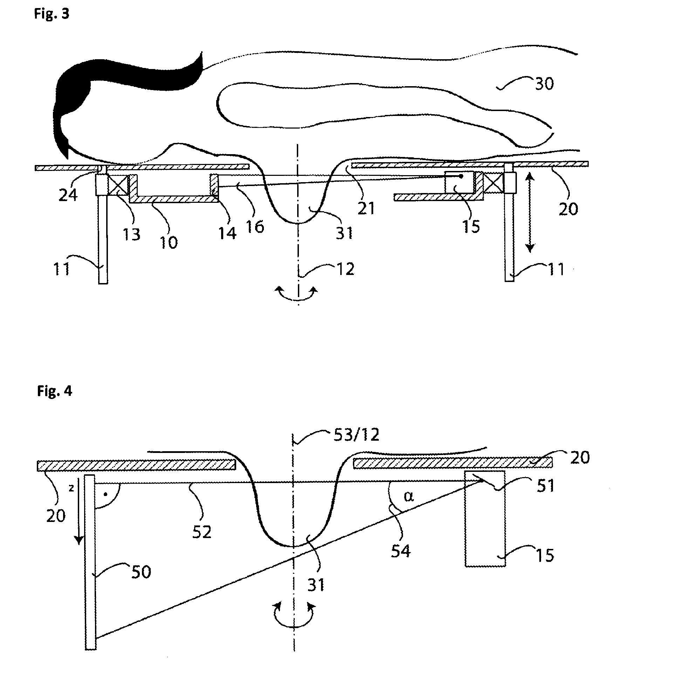

In Figur 3 ist ein seitlicher Schnitt eines erfindungsgemäßen Röntgengerätes dargestellt. Die Patientin 30 ist auf der Patientenliege 20 derart gelagert, dass ihre Brust 31 durch den Brustausschnitt 21 in den Aufnahmebereich einer Gantry 10 hängt. Die Gantry 10 ist eine Spiral-Computertomographen Gantry mit einer Röntgenröhre 15 und einem Detektor 14, welche sich durch das Gantrydrehlager 13 um die Rotationsachse 12 drehen lässt. Gleichzeitig mit der Drehung wird über den Gantryhubantrieb 11 eine Verschiebung in vertikaler Richtung durchgeführt, so dass die Brust spiralförmig abgetastet wird. Der Gantryhubantrieb 11 ist über eine Gantryaufhängung 24 mechanisch steif mit der Patientenliege 20 verbunden.

Figur 4 zeigt schematisch einen Ausschnitt eines erfindungsgemäßen Röntgengerätes zur Erläuterung des Strahlenganges. Die zu untersuchende Brust 31 einer Patientin hängt durch eine Öffnung in einer Auflagefläche 20, welche hier als Patientenliege ausgeführt ist. Selbstverständlich kann diese Anordnung auch um beliebige Winkel gedreht werden, so dass die Patientenliege 20 auch schräg oder senkrecht steht und nur noch als eine Auflagefläche ausgeführt ist, durch welche die Brust hindurch gesteckt ist. Die Brustwand der Patientin soll möglichst nahe an der Patientenliege anliegen, so dass die Brust möglichst vollständig abgebildet werden kann. Zur Abbildung ist eine um die Rotationsachse 12 rotierende Gantry 10 mit einer Röntgenröhre 15 und einem Detektor 50 vorgesehen. Die Röntgenröhre 15 erzeugt ein Strahlenbündel, welches die Brust 31 durchdringt und von dem Detektor 50 aufgenommen wird. Idealerweise ist das Strahlenbündel auf die aktive Fläche des Detektors begrenzt. So soll es keinesfalls die Patientenliege 20 durchdringen. Das Strahlenbündel hat einen Kegelwinkel α (54). Der Zentralstrahl 52, welcher senkrecht auf den Detektor auftrifft, liegt am oberen Rand des Strahlenbündels in der Nähe der Patientenliege 20. Die Zentralachse 53, welche senkrecht zum Zentralstrahl verläuft ist hier identisch mit der Rotationsachse 12 der Gantry.

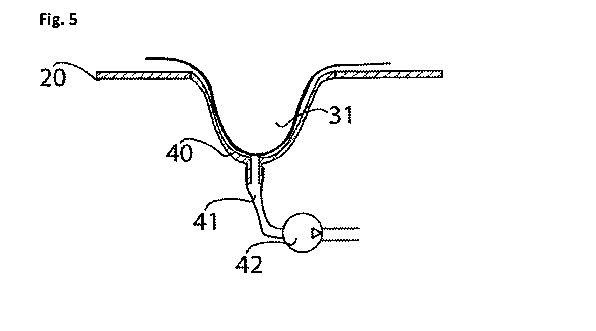

Figur 5 zeigt exemplarisch eine Fixiervorrichtung 40, die fest mit der Patientenliege 20 verbunden ist. Über einen Schlauch 41 ist eine Vakuumpumpe 42 angeschlossen, so dass die Brust 31 der Patientin in der Fixiervorrichtung 40 durch Unterdruck fixiert wird.

### Bezugszeichenliste

- 10: Gantry
- 11: Gantryhubantrieb
- 12: Rotationsachse
- 13: Gantrydrehlager
- 14: Detektor
- 15: Röntgenröhre
- 16: Strahlenfächer
- 20: Patientenliege
- 21: Brustausschnitt
- 22: Patientenliegenhubantrieb
- 23: Räder
- 24: Gantryaufhängung
- 30: Patient
- 31: Brust
- 40: Fixiervorrichtung
- 41: Schlauch
- 42: Vakuumpumpe
- 50: Detektor
- 51: Anode
- 52: Zentralstrahl
- 53: Zentralachse
- 54: Öffnungswinkel

## Patentansprüche

1. Röntgengerät zur Abbildung einer Brust einer Patientin (30), mit
- einer Röntgeneinrichtung umfassend eine Röntgenröhre (15) und einen Röntgendetektor (14), und
- einer näherungsweise horizontal angeordneten Patientenliege (20) mit einem Brustausschnitt (21) zur Aufnahme einer Patientin (30),
wobei eine um eine näherungsweise vertikale Drehachse drehbare Gantry (10)
mit der Röntgenröhre (15) sowie dem Röntgendetektor (14) vorgesehen ist, wobei
- die Gantry (10) an der Patientenliege (20) mechanisch steif aufgehängt ist,
- zur Abbildung der Brust in eine kontinuierliche Rotationsbewegung versetzt werden kann, und
- die Gantry (10) mittels eines Gantryhubantriebs (11) in vertikaler Richtung bewegt werden kann, wobei die vertikale Bewegung in Abhängigkeit von der Rotationsbewegung erfolgt.

2. Röntgengerät nach Anspruch 1,
**dadurch gekennzeichnet, dass**
die Röntgenröhre (15) und der Röntgendetektor (14) derart angeordnet sind, dass der Zentralstrahl (52) des Strahlenfächers (16) der Röntgenröhre an der Seite des Strahlenfächers liegt, welche der Patientenliege zugewandt ist.

3. Röntgengerät nach Anspruch 1,
**dadurch gekennzeichnet, dass**
eine Fixiervorrichtung für die Brust vorgesehen ist, welche an der Patientenliege (20) mechanisch steif aufgehängt ist.

4. Röntgengerät nach Anspruch 1 oder 2,
**dadurch gekennzeichnet, dass**
die Patientenliege (20) durch einen Patientenliegenhubantrieb (22) in der Höhe verstellbar ist.

5. Röntgengerät nach Anspruch 1 oder 2,
**dadurch gekennzeichnet, dass**
die Patientenliege (20) Räder aufweist, um diese zu verschieben.

6. Röntgengerät nach Anspruch 1 oder 2,
**dadurch gekennzeichnet, dass**
die Patientenliege (20) kippbar gelagert ist.

## Claims

1. X-ray machine for imaging a breast of a female patient (30), comprising:
- an X-ray device with an X-ray tube (15) and an X-ray detector (14), and
- an approximately horizontally disposed patient table (20) with a cutout portion (21) for a breast, for accommodating a female patient (30),
wherein a gantry (10) adapted to rotate about an approximately vertical rotation axis is provided with the X-ray tube (15) and the X-ray detector (14), with
- the gantry (10) being rigidly suspended mechanically from the patient table (20),
- being adapted to be set into a continuous rotational movement for imaging a breast, and
- the gantry (10) being adapted to be moved in a vertical direction by means of a gantry lift drive (11), with vertical movement occurring in dependence upon the rotational movement.

2. X-ray machine according to claim 1,
**characterized in that**,
the X-ray tube (15) and the X-ray detector (14) are disposed so that a central beam (52) of a fan beam (16) from the X-ray tube is located on a side of the fan beam facing the patient table.

3. X-ray machine according to claim 1,
**characterized in that**,
a locating device for a breast is rigidly mechanically suspended from the patient table (20).

4. X-ray machine according to claim 1 or 2,
**characterized in that**,
a height of the patient table (20) is adjustable with a patient table lift drive (22).

5. X-ray machine according to claim 1 or 2,
**characterized in that**,
the patient table (20) comprises wheels in order to be moved.

6. X-ray machine according to claim 1 or 2,
**characterized in that**,
the patient table (20) is tiltable.

## Revendications

1. Appareil radiographique pour acquérir des images du sein d'une patiente (30), avec
- un dispositif radiographique comprenant un tube à rayons X (15) et un détecteur de rayons X (14), et
- une couchette (20) approximativement horizontale avec une découpe pour un sein (21), destinée à recevoir une patiente (30),
dans lequel est prévu un bras (10) capable de rotation autour d'un axe approximativement vertical avec le tube à rayons X (15) et le détecteur de rayons X (14),
dans lequel
- le bras (10) est suspendu à la couchette (20) de façon mécaniquement rigide,
- et peut être entraîné dans un mouvement de rotation continu pour acquérir des images du sein,
- le bras (10) peut être déplacé dans le sens vertical au moyen d'un entraînement de levage du bras (11), le mouvement vertical étant effectué en fonction du mouvement de rotation.

2. Appareil radiographique selon la revendication 1, **caractérisé en ce que** le tube à rayons X (15) et le détecteur de rayons X (14) sont disposés de telle façon que le faisceau central (52) des faisceaux en éventail (16) du tube à rayons X se trouve sur le côté de l'éventail dirigé vers la couchette.

3. Appareil radiographique selon la revendication 1, **caractérisé en ce qu'**il est prévu un dispositif de fixation pour le sein qui est suspendu à la couchette (20) de façon mécaniquement rigide.

4. Appareil radiographique selon la revendication 1 ou 2, **caractérisé en ce que** la couchette (20) peut être réglée en hauteur par un entraînement de levage de la couchette (22).

5. Appareil radiographique selon la revendication 1 ou 2, **caractérisé en ce que** la couchette (20) présente des roues permettant de la déplacer.

6. Appareil radiographique selon la revendication 1 ou 2, **caractérisé en ce que** la couchette (20) est supportée de façon à pouvoir basculer.
